Europäisches Patentamt

European Patent Office

Office européen des brevets

(11) Publication number: **0 251 502**
**A1**

## (12) EUROPEAN PATENT APPLICATION

(21) Application number: **87304894.6**

(22) Date of filing: **03.06.87**

(51) Int. Cl.⁴: **A61F 5/448** , A61F 5/44

(30) Priority: **09.06.86 US 872305**

(43) Date of publication of application:
**07.01.88 Bulletin 88/01**

(84) Designated Contracting States:
**AT BE CH DE ES FR GB GR IT LI LU NL SE**

(71) Applicant: **Pfizer Hospital Products Group, Inc.**
**235 East 42nd Street**
**New York New York 10017(US)**

(72) Inventor: **Johns,Owen Louis**
**546, Crystal Drive**
**Madeira Beach Florida(US)**

(74) Representative: **Moore, James William, Dr. et al**
**Pfizer Limited Ramsgate Road**
**Sandwich Kent CT13 9NJ(GB)**

(54) **Low profile ostomy device.**

(57) A low profile ostomy device (100) includes an ostomy bag (102) with an opening (104), a first coupling member (106) attached to the ostomy bag at the opening, and a second coupling member (122) for attachment to a user's body. The first and second coupling members are engaged by the joining of an engaging element (112, 126) on each of the members, the engagement providing a tight mechanical seal between the members within the ostomy bag.

Fig.1.

EP 0 251 502 A1

# LOW PROFILE OSTOMY DEVICE

This invention concerns an improved means for securing an ostomy bag to a user.

Ostomy bags are normally secured to a user's body through a coupling which releasably connects the bag to a pad or dressing adhesively attached to the body. This coupling should allow ready entrance of a stoma into the bag, provide a tight mechanical seal to prevent leakage, and be readily released to assure convenient replacement of the bag. In addition, the bag should be comfortable to the user.

Early ostomy devices, such as that disclosed in West German Auslegeschrift 1105558, provided suitable mechanical seal but lacked means for convenient replacement of the bag. Other ostomy couplings, such as that disclosed in U.S. patent 4,460,363, by the nature of their construction fail to allow the ostomy bag to fit comfortably close to the user's body.

It is, therefore, the primary objective of the present invention to provide an ostomy device which assures ready access for the stoma, tight mechanical seal and convenient replacement of the bag, and also allows for a closer fit of the bag to the body.

The desired objective is accomplished with a low profile ostomy device, which comprises:

(a) an ostomy bag provided with a stoma-encircling opening;

(b) a first coupling member comprising a flat ring portion attached to the ostomy bag with an aperture substantially of the same size as and coincident with the ostomy bag opening, and a first engaging element depending from the ring portion substantially at the aperture and extending into the ostomy bag; and

(c) a second coupling member comprising means for attachment to a user's body, and a second engaging element for engagement with the first engaging element and having an aperture substantially of the same size as the ostomy bag opening, the engagement providing a mechanical seal between the first and second coupling members within the ostomy bag.

In a preferred emlodiment, the first engaging element is in the form of a channel with two sides and a bottom, and the second engaging element is in the form of a flange. The channel bottom may comprise a rib adapted to engage a groove in the flange which splits the flange into two parts. In such case, the flange is preferably formed of a flexible, resilient material and the rib in the first engaging element is slightly wider than the groove in the second engaging element to thereby force the split flange of the second engaging element against the channel sides of the first engaging element when the first and second engaging elements are engaged.

To further improve the mechanical seal, the channel of the first engaging element may have an inner wall with at least one protrusion on the wall, and the split flange has at least one protrusion which interlocks with the wall protrusion when the first and second engaging elements are engaged.

With the present ostomy device, the first coupling member is conveniently heat sealed to the ostomy bag; the ring portion and the first engaging element of the first coupling memter may be integral; the ring portion of the first coupling member may comprise at least one radially extending tab adapted to be gripped by the user; and the attaching means of the second coupling member preferably comprises a layer of adhesive. The first and second coupling members of the ostomy device are readily formed of polyethylene.

The novel features and advantages of the present invention will become apparent from a reading of the following detailed description in conjunction with the accompanying drawings wherein:

FIG. 1 is an elevational view of the first coupling member and a portion of the attached ostomy bag of a preferred embodiment of the present ostomy device, looking in a direction outwardly from the user;

FIG. 2 is a side elevational view of the first coupling member shown in FIG. 1;

FIG. 3 is an enlarged cross-sectional view of the first coupling member only taken along the line 3-3 of FIG. 1;

FIG. 4 is an elevational view of the second coupling member of the present ostomy device, looking in a direction toward the user;

FIG. 5 is a side elevational view of the second coupling member shown in FIG. 4;

FIG. 6 is an enlarged cross-sectional view taken along the line 6-6 of FIG. 4;

FIG. 7 is an exploded cross-sectional view of the first coupling member taken along the line 7-7 of FIG. 1 and of the second coupling member taken along the line 7-7 of FIG. 4; and

FIG. 8 is an enlarged cross-sectional view taken along the line 3-3 of FIG. 1 and line 6-6 of FIG. 2 when the first and second coupling members are fully engaged.

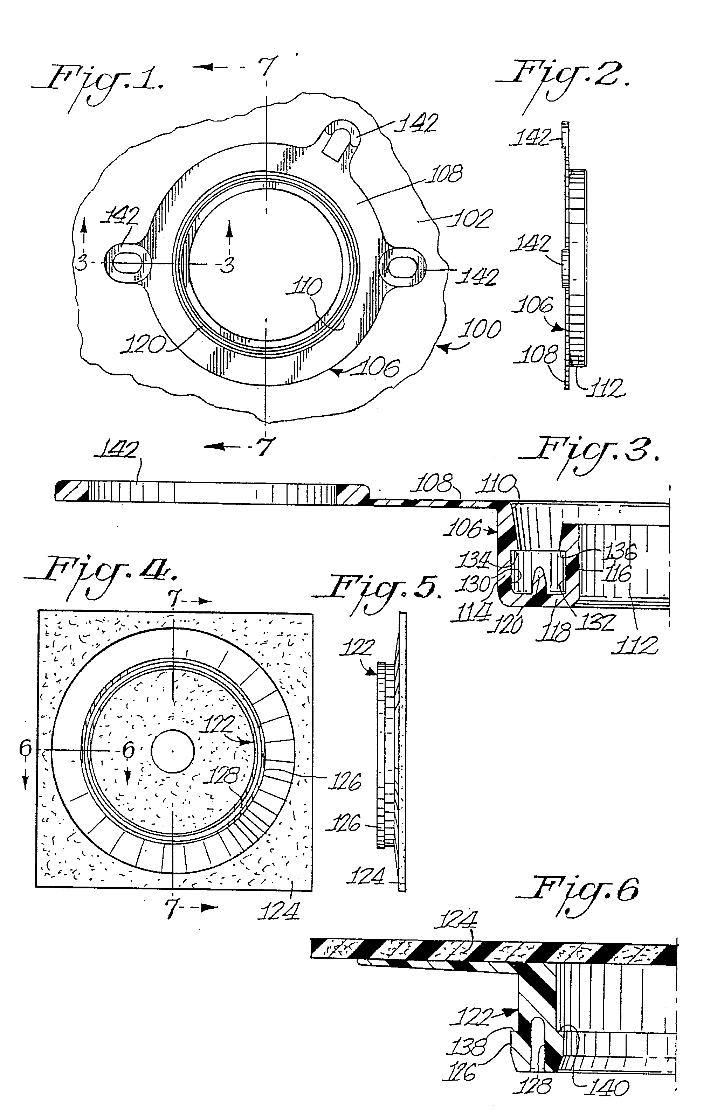

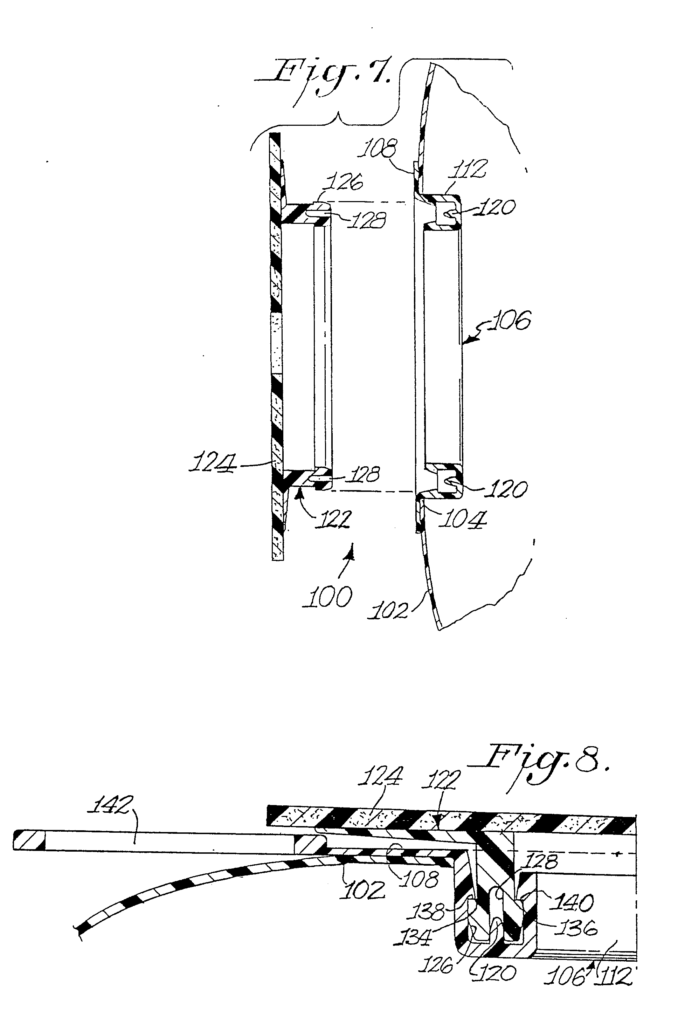

A preferred embodiment of the low profile ostomy device of the present invention is shown in FIGS. 1-8.

This device 100 comprises an ostomy bag 102 having an opening 104 of a size suitable for encircling a user's stoma (not shown). The ostomy bag 102 may be of various sizes and shapes and constructed of various materials, as is well known in the art. Normally, ostomy bag 102 will be constructed from plastic sheet such as polyethylene and will be of flat design to minimize its protrusion from the user's body. Opening 104 is normally in the upper portion of ostomy bag 102, and would be available in various sizes depending on the size of the user. While opening 104 may be of any shape, it will normally be circular.

A first coupling member 106 shown in FIGS. 1-3 comprises a flat ring portion 108 which is attached to the ostomy bag 102 by heat sealing as shown in FIG. 8. Ring portion 108 has an aperture 110 substantially of the same size and shape as and coincident with opening 104. A first engaging element 112, which is integral with ring portion 108 as shown in FIG. 3, depends from ring portion 108 at aperture 110 into ostomy bag 102. Engaging element 112 is in the form of a channel with a long side 114, a short side 116 and a bottom 118. A rib 120 on channel bottom 118 extends into channel 112 substantially parallel to and equidistant from channel sides 114,116.

A second coupling member 122 shown in FIGS. 4-6 comprises an adhesive layer 124 for attaching second coupling member 122 to a user's body (not shown) and a second engaging element 126 for engagement with first engaging element 112 within ostomy bag 102 as shown in FIG. 8, the engagement providing a mechanical seal between first coupling member 106 and second coupling member 122. Second engaging element 126 is in the form of a flange formed from a flexible, resilient material such as polyethylene and split into two essentially equal parts by groove 128.

Since rib 120 in channel 112 is slightly wider than groove 128, when channel 112 and flange 126 are engaged, the split portions of flange 126 are forced by rib 120 against channel sides 114,116, thereby providing an improved mechanical seal between first and second coupling memlers 106,122. To further enhance the sealing action, channel 112 has on each of inner walls 130,132 a protrusion 134,136 on the portion of wall 130,132 opposite channel bottom 118, and flange 126 has on the outside end of each of the split portions a similar protrusion 138,140. Upon engaging flange 126 with channel 112, flange protrusions 138,140 slide over and snap behind channel protrusions 134,136 to thereby provide a friction fit between flange 126 and channel 112 as shown on FIG. 8.

As shown on FIG. 1, first coupling member 106 may include one or more radially extending tabs 142 as part of ring portion 108 for gripping by the user. Such tab 142 is common in the art.

While other materials may be used, polyethylene is the material of choice for both first coupling member 106 and second coupling member 122.

The present ostomy device, by having the coupling means for securing the ostomy bag to a user within the bag itself, allows an ostomy bag to fit closer to the user and provides the advantage, among others, of a marked improvement in the overall aesthetics of the device.

## Claims

1. A low profile ostomy device (100), comprising an ostomy bag (102) provided with a stoma-encircling opening (104) and a pair of coupling members (106, 122), characterized in that

the first coupling member (106) comprises a flat ring portion (108) attached to the ostomy bag with an aperture (110) substantially of the same size as and coincident with the ostomy bag opening, and a first engaging element (112) depending from the ring portion substantially at the aperture and extending into the ostomy bag; and

the second coupling member (122) comprises means (124) for attachment to a user's body, and a second engaging element (126) for engagement with the first engaging element and having an aperture substantially of the same size as the ostomy bag opening, the engagement providing a mechanical seal between the first and second coupling members within the ostomy bag.

2. The ostomy device of claim 1 wherein the first engaging element is in the form of a channel (112) with two sides (114, 116) and a bottom (118), and the second engaging element is in the form of a flange (126).

3. The ostomy device of claim 2 wherein the channel bottom (118) comprises a rib (120) adapted to engage a groove (128) in the flange (126) which splits the flange into two parts.

4. The ostomy device of claim 3 wherein the flange (126) is formed of a flexible, resilient material and the rib (120) in the channel bottom (18) is slightly wider than the groove (128) in the flange to thereby force the split flange against the channel sides (114, 116) when the first and second engaging elements are engaged.

5. The ostomy device of claim 4 wherein the channel (112) has an inner wall (130, 132) with at least one protrusion (134, 136) on the wall, and the split flange (126) has at least one protrusion (138, 140) which interlocks with the wall protrusion when the first and second engaging elements are engaged.

6. The ostomy device of claim 1 wherein the first coupling memler (106) is heat sealed to the ostomy bag (102).

7. The ostomy device of claim 1 wherein the ring portion (108) and the first engaging element (112) of the first coupling member (106) are integral.

8. The ostomy device of claim 1 wherein the ring portion (108) of the first coupling member (106) comprises at least one radially extending tab (142) adapted to be gripped by the user.

9. The ostomy device of claim 1 wherein the attaching means of the second coupling member (122) comprises a layer of adhesive (124).

10. The ostomy device of claim 1 wherein the first and second coupling members (106, 122) are formed of polyethylene.

*Fig.1.*

*Fig.2.*

*Fig.3.*

*Fig.4.*

*Fig.5.*

*Fig.6.*

Fig.7.

Fig.8.

European Patent Office

# EUROPEAN SEARCH REPORT

. Application number

## DOCUMENTS CONSIDERED TO BE RELEVANT

EP 87304894.6

| Category | Citation of document with indication, where appropriate, of relevant passages | Relevant to claim | CLASSIFICATION OF THE APPLICATION (Int. Cl.4) |
|---|---|---|---|
| X | GB - A - 1 217 406 (PHARMA-PLAST) | 1,6,7,9 | A 61 F 5/448 |
| A | * Page 2, line 65 - page 3, line 91; claims; fig. 1-3 * | 2,4,5 | A 61 F 5/44 |
| | -- | | |
| A | GB - A - 2 173 403 (CRAIG MEDICAL PRODUCTS) | 1-7,9 | |
| | * Page 2, line 50 - page 3, line 40; fig. 1-5 * | | |
| | -- | | |
| D,A | US - A - 4 460 363 (STEER) | 1,2,5-10 | |
| | * Column 3, line 38 - column 4, line 34; fig. 1-6 * | | |
| | ---- | | |

|  | TECHNICAL FIELDS SEARCHED (Int. Cl.4) |
|---|---|
| | A 61 F 5/00 |

The present search report has been drawn up for all claims

| Place of search | Date of completion of the search | Examiner |
|---|---|---|
| VIENNA | 16-09-1987 | TSILIDIS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82